# EUROPÄISCHE PATENTANMELDUNG

(11) **EP 0 856 280 A2**
(43) Veröffentlichungstag der Anmeldung: **05.08.1998**
(21) Anmeldenummer: 98101224.8
(22) Anmeldetag: 24.01.1998
(51) Int. Cl.: A61B 5/14

(54) **Blutentnahmevorrichtung**

(30) Priorität: 03.02.1997 DE 19703921
(71) Anmelder: Sarstedt AG & Co., 51588 Nümbrecht (DE)
(72) Erfinder: Sarstedt, Walter, 51588 Nümbrecht (DE)
(74) Vertreter: Pollmeier, Felix, Dipl.-Ing.

(57) **Zusammenfassung**

Bei einer Blutentnahmevorrichtung, bestehend aus einem Röhrchen und einer Verschlußvorrichtung (1) in der ein Unterdruck vorgegeben oder durch Zurückziehen eines Kolbens während der Blutentnahme erzeugt wird, wird eine Schaumbildung beim Einströmen des Blutes in das Röhrchen verhindert, wenn die Verschlußvorrichtung (1) mit einer Strömungsdrossel (4) ausgebildet ist. Zum gleichzeitig verbesserten Kontakt des Blutes mit einem Präparierungsmittel weist die Verschlußvorrichtung (1) einen Körper, vorzugsweise die Strömungsdrossel (4) selbst, auf, der mit einer Präparierung zur Aufbereitung des Blutes versehen ist.

## Beschreibung

Die Erfindung betrifft eine Blutentnahmevorrichtung, bestehend aus einem Röhrchen und einer mit einer Strömungsdrossel ausgebildeten Verschlußvorrichtung, in der ein Unterdruck vorgegeben oder durch Zurückziehen eines Kolbens während der Blutentnahme erzeugt wird.

Eine Blutentnahmevorrichtung dieser Art, bei der das Röhrchen vor dem Ankoppeln an eine Kanüle evakuiert und damit ein Unterdruck vorgegeben wird, ist durch die US 4 492 634 A bekanntgeworden. Das bereits im Herstellerwerk evakuierte Röhrchen wird durch einen Gummistopfen verschlossen, der mit einer quasi eine drosselnde Funktion übernehmenden, ein übermäßiges Schäumen des Blutes verhindernden Verlängerung versehen ist. Aus der DE 44 02 690 C2 ist die andere Variante bekannt, bei der die Blutentnahmevorrichtung im Röhrcheninneren einen Kolben mit Kolbenstange besitzt. Dieser Kolben läßt sich bei Bedarf in eine hintere Rastposition ziehen oder bei bereits angekoppelter Doppelkanüle simultan zum eintretenden Butfluß nach hinten ziehen; in beiden Fällen wird im Inneren des Röhrchens durch das Zurückziehen des Kolbens ein Unterdruck erzeugt.

Zwar läßt sich mittels der bekannten Verschlußvorrichtung der durch das Vakuum bzw. den Unterdruck ansonsten ungebremste Fluß des Blutstromes aus der Kanüle in das Röhrchen drosseln und damit eine starke Schaumbildung vermeiden, jedoch treten Nachteile dann auf, wenn das Röhrchen mit einer meist am Röhrchenboden vorgesehenen Präparierung zur Aufbereitung des Blutes vorbereitet ist. Das in das Röhrchen eingesogene bzw. eintretende Blut kommt dann nämlich erst sehr spät in Kontakt mit dieser Präparierung. Um eine homogene Gerinnungshemmung des Blutes oder eine möglichst frühzeitige Gerinnungsförderung zu erreichen, wird daher angestrebt, die Blutprobe im Röhrchen möglichst rasch nach der Entnahme zu mischen. Bei der praktischen Blutentnahme wird der notwendige Mischvorgang oftmals nicht frühzeitig oder nicht sorgfältig genug durchgeführt, teilweise wird er sogar ganz vergessen.

Bei einer aus der DE 195 19 886 A1 bekannten Blutentnahmevorrichtung anderer Art ist es bekannt, in dem das Blutentnahmeröhrchen verschließenden Stopfen ein Gefäß zur Lagerung und Ausgabe eines Additivs für die Blutanalyse unbeweglich anzuordnen. Das Additiv-Gefäß ist mit einer Abdeckung versehen, die ebenso wie der Stopfen von einer mit einer Blutquelle verbundenen Kanüle punktiert wird. Die Kanüle wird danach teilweise in das Gefäß zurückgezogen, wobei sie ein von der Punktierung herrührendes Loch in der Abdeckung hinterläßt. Durch die Druckdifferenz wird Blut in das Gefäß eingezogen, wo es sich mit dem Additiv vermischt und dieses in den Behälter mitnimmt. Es wird dort ein in dem Gefäß stattfindendes starkes Wirbelmischen von Additiv und Blut angestrebt, was allerdings zu mit Vibrin durchsetzten Schaumgebilden führen kann, die sowohl bei der manuellen als auch in Analysengeräten durchgeführten Serumentnahme stören und deshalb vor der weiteren Bearbeitung entfernt werden müssen, was wegen der erforderlichen Sorgfalt einen hohen Zeitbedarf erfordert und zudem eine erhebliche Kontaminationsgefahr darstellt.

Der Erfindung liegt daher die Aufgabe zugrunde, eine gattungsgemäße Blutentnahmevorrichtung zu schaffen, bei der diese Nachteile nicht auftreten.

Diese Aufgabe wird erfindungsgemäß dadurch gelöst, daß die Strömungsdrossel der Verschlußvorrichtung als Träger einer Präparierung zur Aufbereitung des Blutes dient. Hierbei wurde erkannt, daß auf einen separaten Einsetskörper ganz verzichtet werden kann, wenn die Strömungsdrossel selbst sogleich als Träger des Präparierungsmittels dient. Indem die Verschlußvorrichtung erfindungsgemäß schon gleich nicht nur mit einem geeigneten Drosselmittel, sondern auch als Präparierungsträger ausgebildet ist, läßt sich mit einem gegebenenfalls einheitlichen bzw. -stückigen Teil ein Doppeleffekt erreichen. Es wird nämlich einerseits aufgrund der Drosselwirkung bei gleichzeitiger Umlenkung und Verteilung des Blutes die Strömungsgeschwindigkeit auf einen unschädlichen Wert verringert und damit die Schaumbildung unterdrückt sowie andererseits auch für einen frühzeitigen und ungehinderten Kontakt des entnommenen Blutes mit einem geeigneten Präparierungsmittel gesorgt.

Weitere Einzelheiten und Vorteile der Erfindung ergeben sich aus dem Patentanspruch und der nachfolgenen Beschreibung.

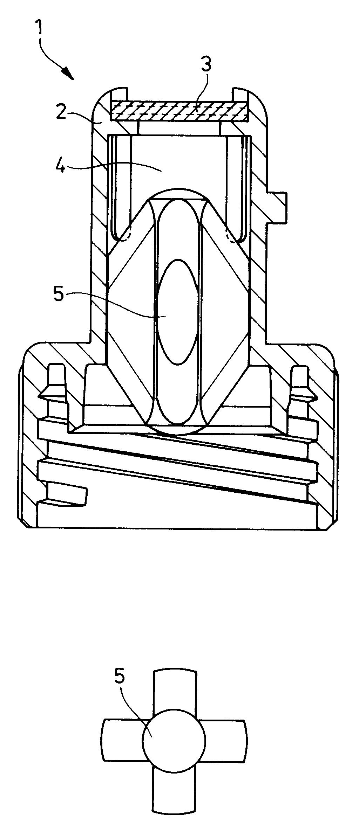

Die einzige Figur zeigt beispielsweise eine Verschlußvorrichtung 1 einer Blutentnahmevorrichtung, welche auf ein Röhrchen (nicht gezeigt) aufgeschraubt wird. Die Verschlußvorrichtung 1 besitzt einen Dom 2, in den eine luftundurchlässige Membane 3 eingeschweißt ist. Bei einer Blutentnahme wird das vordere Ende einer Doppelkanüle (nicht gezeigt) in ein Blutgefäß gestochen. Anschließend wird die Röhre mit dem Dom 2 auf das hintere Ende der Kanüle aufgeschoben. Dabei durchsticht die Kanüle die Membrane 3, so daß eine Strömungsverbindung zwischen der Vene oder Arterie eines Patienten und dem Röhrcheninnenraum hergestellt wird. Durch den im Röhrchen beispielsweise vorliegenden Unterdruck wird das Blut eingesaugt.

Um eine Verringerung der Strömungsgeschwindigkeit zu erzielen, wird im gezeigten Ausführungsbeispiel in dem Dom 2 eine Strömungsdrossel 4 in Form eines z.B. kreuzförmigen Teiles 5 angeordnet, die das Blut nach Austritt aus der Kanüle gleichmäßig verteilt und dessen Geschwindigkeit verringert. Das im gezeigten Ausführungsbeispiel die Strömungsdrossel 4 darstellende Einlegeteil 5 ist mit einer Präparierung versehen, die durch die große, allseits von Blut umspülte Oberfläche direkt mit dem Blut in Kontakt gelangt, so daß die Strömungsdrossel 4 gleichzeitig eine gleichmäßige Verteilung einer beispielsweise gerinnungshemmenden oder -fördernden Substanz im Blut ermöglicht.

## Patentansprüche

1. Blutentnahmevorrichtung, bestehend aus einem Röhrchen und einer mit einer Strömungsdrossel ausgebildeten Verschlußvorrichtung, in der ein Unterdruck vorgegeben oder durch Zurückziehen eines Kolbens während der Blutentnahme erzeugt wird,
**dadurch gekennzeichnet,**
daß die Strömungsdrossel (4) der Verschlußvorrichtung (1) als Träger einer Präparierung zur Aufbereitung des Blutes dient.
